Europäisches Patentamt

**European Patent Office**

Office européen des brevets

(19)

(11) Publication number: **0 213 056 B1**

(12) # EUROPEAN PATENT SPECIFICATION

(45) Date of publication of patent specification: **02.05.91**  (51) Int. Cl.⁵: **A61M  15/00,** B05B 17/06

(21) Application number: **86630122.9**

(22) Date of filing: **07.08.86**

(54) **Method and apparatus for producing a stream of heated vapor particularly useful for therapeutic purposes.**

(30) Priority: **15.08.85 CA 488819**

(43) Date of publication of application:
**04.03.87 Bulletin  87/10**

(45) Publication of the grant of the patent:
**02.05.91 Bulletin  91/18**

(84) Designated Contracting States:
**AT BE CH DE FR GB IT LI NL SE**

(56) References cited:
**DE-A- 2 154 167**
**US-A- 3 469 785**
**US-A- 3 593 712**

(73) Proprietor: **RHINOTHERM NETZER SERENI
LIMITED PARTNERSHIP
Kibbutz Netzer Sereni
Doar Beer Yaacov 70395(IL)**

(72) Inventor: **Verity, Nigel
30 St. Charles Avenue
Dorval Quebec H95 3T3(CA)**

(74) Representative: **Weydert, Robert et al
OFFICE DENNEMEYER S.à.r.l. P.O. Box 1502
L-1015 Luxembourg(LU)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an apparatus for producing a stream of heated vapor according to the preamble of claim 1. The invention is particularly useful for therapeutic purposes, and is therefore described below with respect to this application.

It has recently been shown that the application of a stream of heated vapor to the nasal passages can have a beneficial therapeutic effect on persons suffering from a common cold and other similar ailments such as sinusitis, allergic and non-allergic rhinitis, nasal polyps, asthma and hay fever. Several patents, for example US-A-4,369,777 and 4,401,114, have issued describing this treatment, and machines are now commercially available for providing this treatment. However, while the treatment has been found to be very effective, the machines now in use are large and noisy, tend to overheat at the entrance to the nostril, tend to produce considerable water splash, are frequently unreliable, and are very expensive.

US-A-3,469,785 discloses an apparatus for producing a stream of vapor comprising a chamber adapted to receive a quantity of liquid to be vaporized; an ultrasonic generator disposed within the chamber to be submerged within the liquid when received therein; drive means for driving the ultrasonic generator to produce a spout of intensely-agitated liquid spouting upwardly out of the surface of the liquid in the chamber; gas feeding means for feeding a gas into the chamber; and a delivery tube connected to the chamber for outletting vapor therefrom in the form of a confined stream of gas saturated with the vapor and having a small quantity of liquid droplets mixed therein.

In the apparatus described in US-A-3,469,785, the ultrasonic generator is disposed substantially in the horizontal plane so that the intensely-agitated liquid spouts substantially vertically out of the surface of the liquid in the chamber. Air is admitted into the liquid chamber and is circumferentially swirled therein. The fall-back of droplets from the spout into the liquid pool disturbs the formation of the spout, and thereby decreases the nebulizing capacity of the device.

In US-A-3,901,443 the ultrasonic generator is mounted at an inclination of 2-22° with respect to the surface level of the liquid in order to minimize the disturbance to the formation of the spout by the fall-back of the droplets from the spout into the liquid pool. US-A-4,410,139 provides a partition surrounding the spout in order to separate the larger non-vaporized particles from the spout base to reduce the disturbance to the formation of the spout at the spout base. In EP-A-0 125 210 hot air is blown through a porous member having an end immersed in a liquid.

The object of the present invention is to provide an improved apparatus for nebulizing a liquid in a manner which substantially increases the nebulizing capacity and also minimizes the disturbance to the formation of the spout by the fall-back of droplets from the spout into the liquid pool.

To achieve this, the apparatus for producing a stream of heated vapor according to the invention is characterized in that the gas feeding means feeds the gas into the chamber in the form of a confined stream of hot gas impinged directly on the liquid spout to impart an arcuate trajectory to the spout.

Such an arrangement has been found to increase the nebulization from the spout by the increased area of the spout contacted by the hot gas because of the arcuate trajectory of the spout, and by the reduced disturbance to the formation of the spout at the spout base because of the shifting laterally, with respect to the spout base, of the fall-back into the pool of larger liquid droplets from the spout.

The apparatus is particularly applicable wherein the liquid is water, e.g. distilled water or saline water, and the gas is air.

Apparatus constructed in accordance with the above features has been found to provide significant beneficial therapeutic effects on persons suffering from the common cold and other nasal elements. In addition, such apparatus operates with very little noise, and provides a substantially uniform temperature from the hyper-evaporation chamber to the entrance to the user's nostrils, thereby minimizing the possibility of irritating or damaging the person's nostrils by an unduly high temperature. It has also been found that a significantly higher temperature can be achieved in the stream reaching the nasal mucosa, without undue discomfort to the user. Further, the stream of vapor is sufficiently moist to keep the nasal tissues moist, and thereby to minimize irritation or damage, but not so moist as to produce water splash. Still further, such apparatus has been found to be very reliable and significantly less expensive than the apparatus now in use.

Additional features and advantages of the apparatus for producing a stream of heated vapor will be apparent from the description of the accompanying drawings, wherein:

Fig. 1 is a three dimensional view illustrating one form of apparatus for producing a stream of heated vapour;

Fig. 2 is a front elevational view of the apparatus of Fig. 1;

Fig. 3 is a three dimensional view illustrating the apparatus of Fig. 1 from the opposite side;

Fig. 4 is a view along lines IV--IV of Fig. 3;

and Fig. 5 is a fragmentary view illustrating a variation.

The apparatus illustrated in the drawings comprises a housing, generally designated 2, of appropriate material such as plastic, supported on rubber feet 3. Housing 2 includes a partition 4 extending the full vertical height of the housing and dividing its rear end into two compartments 6 and 8 each extending the complete vertical height of the housing. A rotary pump, generally designated 10, is disposed within compartment 6 and pumps air to a duct 12 disposed within compartment 8.

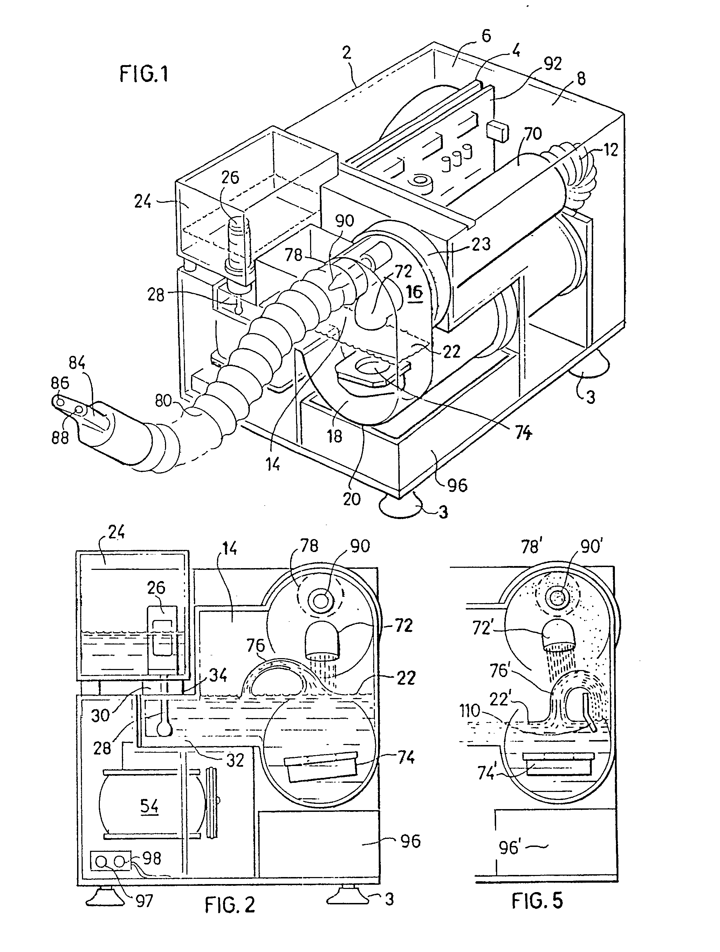

Communicating with duct 12 is a chamber 14 located at the front side of the apparatus and defined by a vertical rear wall 16 separating the chamber from compartment 8, a front vertical wall 18, a bottom curved wall 20, and a top curved wall 23. Compartment 14 is adapted to receive a quantity of a liquid, namely water in the described preferred embodiment, to a level indicated at 22 in Fig. 1.

The water introduced into compartment 14 is supplied from a water reservoir 24 constructed as a separate container and sealed from the atmosphere except for a valve assembly, generally designated 26, having a depending operator stem 28. Valve assembly 26 is of a known construction and is normally closed, but is automatically opened by its operator stem 28 passing through an inlet port 30 in housing 2 and engageable with a ledge 32 formed in the housing. When reservoir 24 is so applied to housing 2, thereby opening valve 26, the water is automatically fed from reservoir 24 through port 30 into chamber 14 to the level 22, namely the level of the lower face of housing wall 34 formed with the inlet port 30. As also known in valve assemblies of this type, the water is automatically maintained at level 22. Such valves are commonly used in kerosene lamps and the like.

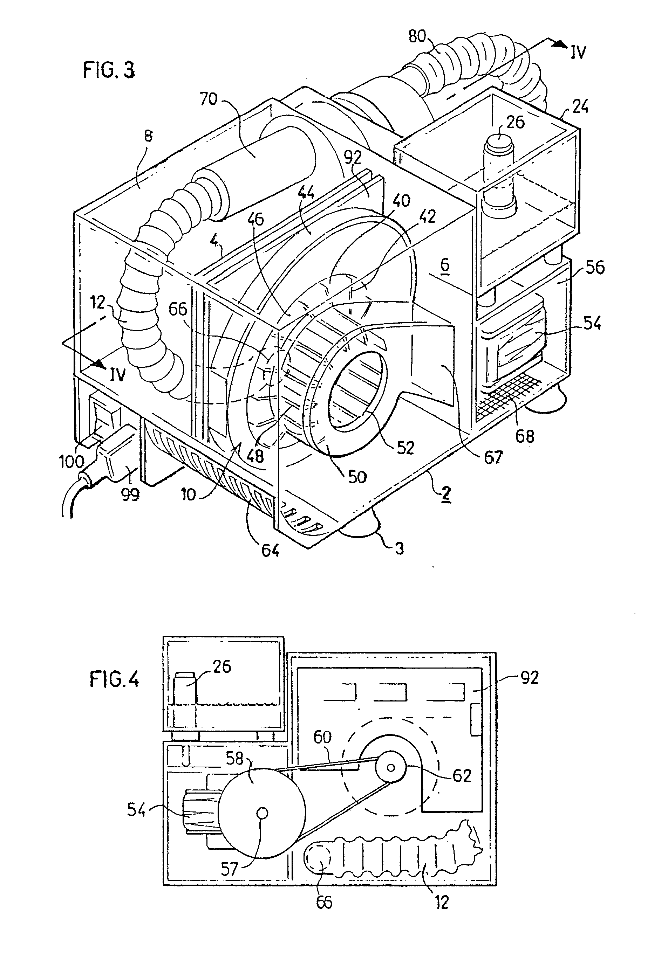

As shown particularly in Fig. 3, pump 10 disposed within compartment 6 is of the rotary type, including a disc 40 formed with a circular array of radially-extending vanes 42 rotatably mounted within a housing 44 fixed to partition 4. Housing 44 is formed with a central opening 46, and vanes 42 include extensions 48 extending through opening 46 into extension 50 of housing 44. Extension 50 is also formed with a central opening 52 communicating with the interior of the pump compartment 6.

Rotary pump 10 is rotated by an electric motor 54 disposed within a compartment 56 at the front of the apparatus just underlying the liquid reservoir 24. As shown in Fig. 4, motor 52 drives a shaft 57 having a pulley wheel 58 coupled by a pulley belt 60 to another pulley wheel 62 secured to rotatable disc 40 of pump 10. Motor 54 is preferably a shaded-pole induction motor operating at a rotational speed of 3000 RPM when supplied from a 60 cycle power source, and the transmission ratio between pulleys 58 and 62 is such that rotary disc 40 is driven at a speed of 6000 RPM.

Air is inletted into pump compartment 6 via a grill section 64 (Fig. 3) integrally formed with housing 2 at its lower rear end. A porous filter, such as of foam rubber (not shown), may be supported on grill 64 to filter the air inletted into compartment 6. A portion of the air is pumped by pump 10 through an outlet opening 66 formed at the lower end of partition 4 into conduit 12 for conduction by the conduit to the hyper-vaporization chamber 14 at the front end of the apparatus. Another portion of air is pumped by pump 10 via an opening 67 into compartment 56 for cooling the electric motor 54. The latter compartment is also integrally formed with a grill 68 for exhausting this cooling air.

Conduit 12, conducting the air pumped by pump 10 into chamber 14, includes an electrical heater 70 which heats the air passing therethrough so that the air exiting from conduit 12 into chamber 14 is at a high temperature. This air exits from the conduit into chamber 14 via an outlet nozzle 72 oriented so as to direct the heated air downwardly into chamber 14.

Chamber 14 includes an ultrasonic generator 74, in the form of a piezoelectric crystal, disposed within the chamber so as to be submerged by the liquid when received therein to the level 22. Piezoelectric crystal 74 may be of the type commonly used in ultrasonic humidifiers. Preferably, it is operated at a voltage of 120 volts (peak-to-peak) and at a frequency of 1.6 mHz, and it agitates the liquid within chamber 14 such as to produce a spout of intensely-agitated liquid spouting upwardly, as shown at 76, out of the liquid surface and falling back by gravity to the liquid surface.

Such spouts are also formed in ultrasonic humidifiers using piezoelectric crystals. As distinguished from ultrasonic humidifiers, however, in the present invention a confined stream of hot air, as generated by pump 10 and heated by heater 70, is directed by a conduit 12 and its outlet 72 to impinge the liquid spout 76. This produces an instant vaporization of liquid within the spout 76 so that the air within chamber 14 is completely saturated, i.e. of 100% humidity, and also includes a quantity of water droplets having an average diameter of 4-8 microns. In addition, the pressure within chamber 14 is increased 5-20 cm (water), preferably 10 cm, above atmospheric pressure, which is at least one order of magnitude above the pressure increase (about 0.5 cm) in a conventional humidifier.

Chamber 14 is provided at its upper end with an outlet 78 connected to one end of a flexible delivery tube 80. The opposite end of delivery tube 80 is closed by a cap 84 formed with a pair of parallel, restricted passageways 86, 88, spaced so as to be alignable with the two nostrils of a user of the apparatus. Outlet 78 and delivery tube 80 thus produce a confined stream of heated air of 100% humidity and including a quantity of water droplets of very small diameter, which stream is split into two streams by passageways 86, 88. The two streams enter the two nostrils of the user with sufficient pressure, e.g. 5-20 cm (water), to reach the nasal mucosa without inhalation by the user.

The illustrated apparatus further includes a heat sensor 90 (Figs. 1, 2) at the outlet end of chamber 14 to measure the temperature thereat. Heat sensor 90 controls heater 70 to maintain a relatively constant temperature at the outlet of chamber 14. Since the air within the chamber is 100% humidified, as described above, there is very little temperature drop in the passage of the heated water vapor stream from chamber 14 via delivery tube 80 to the nostrils of the user.

The temperature of the heated water vapor exiting from chamber 14 is preferably within the range of 40-55° C; particularly good results have been obtained when this temperature is 49° C. The delivery tube 80 should be at least 20 cm in length, preferably about 35 cm, which produces a temperature drop of approximately 1.5° C. Another temperature drop of 1-3° C may occur during the passage of the vapor stream to the nasal mucosa, depending on whether cap 84 is held against the nostrils, which is comfortably permitted in the illustrated device, or spaced slightly (e.g. 1 cm) therefrom. As described above, heat sensor 90 at the outlet of chamber 14 maintains this temperature relatively constant, and the temperature drop during the travel of the heated stream of air via delivery tube 80 to the nostrils of the user is very low because the heated air is 100% humidified by the above-described technique. Heater 70 is preferably operated to produce a temperature of 250-400° C, preferably 300° C in the stream of air exiting from nozzle 72 into chamber 14.

The water content of the 100% humidified air outletted from chamber 14 via delivery tube 80 is from 75-90% of the total water content of the stream exiting from that chamber; that is, the water droplet content is from 25-10% of the total water content of this stream. The purpose of the water droplets is to maintain the tissues moist and thereby to prevent irritation or damage. If the water droplet content is less than 10%, it has been found that this unduly irritates the tissues and could even cause damage; whereas if the water droplet content is more than 25%, this makes the treatment less effective and also overly wets the tissues so as to cause water to drip from the nostrils. Best results have been obtained when the total water content of the outletted stream is 80% in the humidified air and 20% in the water droplets. The described apparatus also enables the use of saline water, which is not possible with the existing machines.

The electrical circuit for operating the pump motor 54, heater 70 and the piezoelectric crystal 74 has not been shown, as conventional circuitry may be used for this purpose. Preferably, the components of the electical circuit are carried by a printed circuit board 92 (Fig. 1) directly mounted to partition 4, which partition also mounts the rotary disc 40 of pump 10. Partition 4 is made of aluminum sheet material in order to act as a heat sink for the heat generated by the electrical components mounted on printed circuit board 92.

Cap 84 at the end of delivery tube 80 is preferably removable and replaceable by another cap when the apparatus is to be used by another person. Also, an open-top container 96 is supported by housing 2 to underlie chamber 14 to catch any water drippings, e.g., occurring when the water reservoir 24 is applied. Cap 84, chamber 14, reservoir 24 and delivery tube 80 are all removable for washing.

In Fig. 2, piezoelectric crystal 74 is mounted at a slight incline to the vertical axis of chamber 14. Thus, the liquid spout 76 exiting from the surface 22 of the liquid is also at a slight incline to the vertical axis of the chamber, producing a "plume" like appearance. Outlet 72, for the hot air delivered to chamber 14 by conduit 12, is directed downwardly over the spout to produce a concentrated jet impinging the spout about 1 cm from its bottom.

The front wall of housing 2 includes one or more light indicators for indicating various conditions. Thus, light indicator 97 indicates whether the apparatus is operating, and light indicator 98 indicates a possible malfunction, such as overheating (or underheating) of the heated vapor stream exiting from chamber 14. The apparatus further includes a plug 99 for connecting same to the supply mains, and a switch 100 for turning the apparatus "on" and "off".

Fig. 5 illustrates a variation, wherein the piezoelectrical crystal 74' is mounted precisely along the vertical axis to produce a vertical spout 76', but nozzle 72' is tilted at a slight angle to the vertical axis so as to deflect the spout towards a divider wall 110 laterally of the spout. The humidified air and fine water droplets exit via the outlet 78' (where sensor 90' is located), whereas the larger water droplets fall back on the other side of wall 110 into the water pool.

The apparatus illustrated in the drawings operates as follows:

Removable reservoir 24 is first filled with water. The dimensions of this reservoir are such that it contains a quantity of water sufficient for a standard one-half hour treatment. For filling the reservoir, it is detached from housing 2 and filled through its valve assembly 26 as known in such valve assembly constructions. The reservoir is then inverted to the position illustrated in the drawings and is applied to the upper end of housing 2, with stem 28 of the valve assembly passing through opening 30 in housing wall 34 until the stem engages ledge 32 of the housing. When this occurs, the valve opens and permits the water within it to flow through opening 30 into chamber 14 where it automatically assumes the level indicated at 22 in Fig. 3. This level, which is even with the bottom face of wall 34 in which inlet opening 30 is formed, is automatically maintained during the operation of the apparatus by valve 26 as known in valves of such construction.

Motor 54 is then energized to rotate pump disc 40 via pulley wheels 58, 62 and pulley 60. As indicated earlier, disc 40 is preferably rotated at approximately 6000 RPM. Heater 70 is then energized. A portion of the air inletted into compartment 6 via grill openings 64 is pumped by the vanes on rotary disc 40 into conduit 12 where it is heated by heater 70 and is then directed via outlet 72 downwardly into chamber 14 containing the water to be vaporized. Another portion of the pumped air is passed via opening 67 into compartment 56 to cool motor 54, this part of the air being exhausted via grill openings 68.

At the same time that pump motor 54 and heater 70 are energized, piezoelectric crystal is also energized so as to vibrate at a frequency of 1.6 mHz. As known in conventional humidifiers, this produces in the water within chamber 14, a spout of intensely-agiated water which spouts upwardly, as shown at 76, out of the liquid surface and then falls back by gravity into the water. The water within spout 76, constituted of a multitude of highly agitated small droplets of water, is impinged by the stream of hot air outletted from outlet 72 of conduit 12; this hot air instantly vaporizes a portion of the liquid within the spout. The result is that the vapor within chamber 14 is constituted of 100% humidified air containing a small quantity of liquid droplets having an average diameter of 4-8 microns.

The interior of chamber 14 is also pressurized by pump 10 to a pressures 5-20 cm (water) above atmospheric, so that the vapor within chamber 14 is outletted from outlet 78 into delivery tube 80 in the form of a confined stream of the hot 100% humidified air.

This stream of heated water vapor and water droplets passes through the flexible delivery tube 80 to the cap 82 at the end of the tube. This end is held in contact with, or slightly spaced (no more than 1 cm) from, the user's nose, with openings 86, 88, aligned with the user's nostrils. The heated water vapor exiting from the end of delivery tube 80 is pressurized 5-20 cm (water) above atmospheric, and therefore the heated water vapor passes into the user's nostril at sufficient velocity to reach the nasal mucosa without inhalation by the user since inhalation is frequently difficult or impossible when the user is suffering from a common cold.

## Claims

1. Apparatus for producing a stream of heated vapor, comprising a chamber (14) adapted to receive a quantity of liquid to be vaporized; an ultrasonic generator (74) disposed within said chamber (14) to be submerged by the liquid when received therein; drive means for driving said ultrasonic generator (74) to produce a spout (76) of intensely-agitated liquid spouting upwardly out of the surface (22) of the liquid in the chamber; gas feeding means (12) for feeding a gas into said chamber (14); and a delivery tube (80) connected to said chamber (14) for outletting vapor therefrom in the form of a confined stream of gas saturated with said vapor and having a small quantity of liquid droplets mixed therein; characterized in that said gas feeding means (12) feeds said gas into said chamber in the form of a confined stream of hot gas impinged directly on said liquid spout (76) to impart an arcuate trajectory to the spout.

2. The apparatus according to Claim 1, wherein said gas feeding means for feeding a stream of hot gas to impinge said liquid spout (76) comprises:
an air pump (10) pumping said gas via a conduit (12) into said chamber;
and a heater (70) enclosing said conduit (12) so as to heat the gas flowing therethrough from the air pump (10) into said chamber (14).

3. The apparatus according to Claim 2, wherein said air pump (10) is a rotary pump effective to pressurize said chamber (14) to a pressure of 5-20 cm of water above atmospheric pressure.

4. The apparatus according to Claim 1, further including a reservoir (24) for the liquid, and valve means (26) for automatically controlling the flow of said liquid from the reservoir (24) to said chamber (14) so as to maintain a substantially constant level (22) in said chamber (14).

5. The apparatus according to Claim 4, wherein said apparatus includes a housing (2) defining said chamber (14), said reservoir (24) being a separate container receivable on said housing and including a normally closed valve (26) having a valve operator (28) automatically opening said valve to cause the liquid in said reservoir (24) to flow into said compartment (14) upon the application of the reservoir (24) to said housing (2).

6. The apparatus according to Claim 1, wherein said gas feeding means for feeding a stream of hot gas to impinge said liquid spout (76) in said chamber (14) comprises a pump (10) driven by a rotary electric motor (54) for pumping said gas via a conduit (12) into said chamber, and a heater (70) enclosing said conduit (12) to heat the gas flowing therethrough from the pump (10) into said chamber (14), said housing (2) including partition walls (4) defining a first compartment (6) for said pump, and a second compartment (8) for said motor.

7. The apparatus according to Claim 1, wherein said delivery tube (80) is flexible and its opposite end is provided with a cap (84) having a pair of passageways (86, 88) to split the heated stream into two streams for application to the two nostrils of a user.

8. The apparatus according to Claim 1, wherein said ultrasonic generator (74) is a piezoelectric crystal.

9. The apparatus according to Claim 1, wherein said gas feeding means for feeding a stream of hot gases to impinge said liquid spout (76) in the chamber (14) comprises a rotary pump (10) pumping said gas via a conduit (12) into said chamber (14), and a heater (70) enclosing said conduit (12) to heat the gas flowing therethrough from the pump (10) into said chamber (14); and wherein the chamber outlet (78) includes a temperature sensor (90) for sensing the temperature thereat and for automatically controlling said heater (70) to maintain a relatively constant temperature at said chamber outlet (78).

**Revendications**

1. Dispositif de production d'un jet de vapeur chaude, comprenant une chambre (14) adaptée pour recevoir une quantité de liquide à transformer en vapeur, un générateur d'ultrasons (74) disposé à l'intérieur de ladite chambre (14) de manière à être submergé dans le liquide lorsqu'il y est reçu, des moyens de commande pour amener le générateur à ultrasons (74) à produire un jet (76) de liquide sous agitation intense jaillissant en hauteur hors de la surface (22) du liquide dans la chambre, des moyens d'alimentation (12) en gaz pour amener un gaz dans ladite chambre (14), et un tuyau de sortie (80) relié à ladite chambre (14) pour en faire sortir la vapeur sous forme d'un flux confiné de gaz saturé avec ladite vapeur et mélangé à une petite quantité de gouttelettes liquides, *caractérisé en ce que* les moyens d'alimentation (12) en gaz amènent le gaz dans la chambre sous forme d'un flux confiné de gaz chaud entrant en collision directe avec ledit jet (76) de liquide pour imprimer à ce dernier une trajectoire courbe.

2. Dispositif selon la Revendication 1, dans lequel lesdits moyens d'alimentation en gaz pour amener un jet de gaz chaud en collision avec ledit jet (76) de liquide comprennent:
une pompe (10) à air injectant ledit gaz dans ladite chambre via une canalisation (12);
et un élément chauffant (70) entourant ladite canalisation (12) afin de réchauffer l'air s'écoulant de la pompe (10) à air jusqu'à ladite chambre (14).

3. Dispositif selon la Revendication 2, dans lequel ladite pompe (10) à air est une pompe rotative permettant de pressuriser ladite chambre (14) sous une pression comprise entre 5 et 20cm d'eau au-dessus de la pression atmosphérique.

4. Dispositif selon la Revendication 1, comprenant un réservoir (24) pour le liquide et des moyens de soupape (26) pour contrôler de manière automatique l'écoulement dudit liquide du réservoir (24) à ladite chambre (14) afin de maintenir un niveau sensiblement constant (22) dans ladite chambre (14).

5. Dispositif selon la Revendication 4, dans lequel ledit dispositif comprend un carter (2) définissant ladite chambre(14), ledit réservoir (24) étant un réservoir séparé adptable sur ledit carter et comprenant une soupape (26) normalement fermée ayant une tige de commande (28) ouvrant automatiquement ladite soupape pour faire écouler le liquide dudit réservoir (24) dans ledit compartiment (14) lorsque le réservoir (24) est mis en place sur ledit carter (2).

6. Dispositif selon la Revendication 1, dans lequel lesdit moyens d'alimentation en gaz pour amener un jet de gaz chaud à entrer en collision avec un jet (76) de liquide dans ladite chambre (14) comprennent une pompe (10) entraînée par un moteur électrique rotatif (54) pour injecter ledit gaz dans ladite chambre via une canalisation (12), et un élément chauffant (70) entourant ladite canalisation (12) pour réchauffer le gaz s'y écoulant en allant de la pompe (10) à ladite chambre (14), ledit carter (2) comportant des cloisons (4) définissant une premier compartiment (6) pour ladite pompe et un second compartiment (8) pour ledit moteur.

7. Dispositif selon la Revendication 1, dans lequel ledit tuyau de sortie (80) est flexible et son extrémité opposée est munie d'un capuchon (84) muni de deux passages (86, 88) destiné à partager le jet chaud en deux jets pour les appliquer aux narines d'un utilisateur.

8. Dispositif selon la Revendication 1, dans lequel ledit générateur à ultra-sons (74) est un cristal piézo-électrique.

9. Dispositif selon la Revendication 1, dans lequel lesdits moyens d'alimentation en gaz pour amener un jet de gaz chaud en collision avec ledit jet (76) de liquide dans la chambre (14) comprennent une pompe rotative (10) injectant ledit gaz dans ladite chambre (14) via une canalisation (12) et un élément chauffant (70) entourant ladite canalisation (12) pour réchauffer le gaz s'y écoulant en allant de la pompe (10) à ladite chambre (14) et dans lequel l'orifice de sortie (78) de la chambre comprend une sonde de température (90) pour détecter la température et pour contrôler de manière automatique ledit élément chauffant (70) pour qu'il maintienne une température relativement constante au niveau dudit orifice de sortie (78) de la chambre.

## Ansprüche

1. Vorrichtung zum Herstellen eines Stroms erhitzten Dampfes, mit einer Kammer (14) zum Aufnehmen einer Menge zu verdampfender Flüssigkeit; einem Ultraschallerzeuger (74), der innerhalb der Kammer (14) angeordnet ist, um in die Flüssigkeit eingetaucht zu sein, wenn diese darin aufgenommen ist; einer Antriebseinrichtung zum Antreiben des Ultraschallerzeugers (74), um einen Strahl (76) stark bewegter Flüssigkeit zu erzeugen, der nach oben aus der Oberfläche (22) der Flüssigkeit in der Kammer (14) spritzt; einer Gaszuführeinrichtung (12) zum Zuführen von Gas in die Kammer (14); und einem Ausströmrohr (80), das mit der Kammer (14) verbunden ist, zum Auslassen des Dampfes in Form eines begrenzten Stroms von mit dem Dampf gesättigtem Gas, mit dem eine geringe Menge von Flüssigkeitstropfen vermischt ist; dadurch gekennzeichnet, daß die Gaszuführeinrichtung (12) das Gas in Form eines begrenzten Stroms heißen Gases, der direkt auf den Flüssigkeitsstrahl (76) aufprallt, um dem Strahl eine gekrümmte Bahn zu verleihen, der Kammer zuführt.

2. Vorrichtung nach Anspruch 1, wobei die Gaszuführeinrichtung zum Zuführen eines Stroms heißen Gases zum Aufprallen auf den Flüssigkeitsstrahl (76) aufweist: eine Luftpumpe (10), die das Gas über eine Leitung (12) in die Kammer pumpt; und ein Heizgerät (70), das die Leitung (12) umschließt, so daß es das durch sie hindurch von der Luftpumpe (10) in die Kammer (14) strömende Gas erhitzt.

3. Vorrichtung nach Anspruch 2, wobei die Luftpumpe (10) eine Umlaufpumpe ist zum Erhöhen des Drucks in der Kammer (14) bis zu einem Druck von 5 bis 20 cm Wassersäule über Atmosphärendruck.

4. Vorrichtung nach Anspruch 1, weiter mit einem Behälter (24) für die Flüssigkeit und einer Ventileinrichtung (26) zum Selbstregeln des Stroms der Flüssigkeit aus dem Behälter (24) zu der Kammer (14), um so ein im wesentlichen gleichbleibendes Niveau (22) in der Kammer aufrechtzuerhalten.

5. Vorrichtung nach Anspruch 4, wobei die Vorrichtung ein Gehäuse (2) aufweist, das die Kammer (14) bildet, wobei der Behälter (24) ein getrennter Transportbehälter ist, der auf dem Gehäuse aufnehmbar ist und ein normalerweise geschlossenes Ventil (26) mit einer Ventilbedienungsvorrichtung (28) aufweist, die das Ventil selbsttätig öffnet, damit die Flüssigkeit in dem Behälter (24) in die Kammer (14) fließt, wenn der Behälter (24) auf das Gehäuse (2) aufgesetzt wird.

6. Vorrichtung nach Anspruch 1, wobei die Gaszuführeinrichtung zum Zuführen eines Stroms heißen Gases zum Aufprallen auf den Flüssigkeitsstrahl (76) in der Kammer (14) eine Pumpe (10) aufweist, die durch einen elektrischen Umlaufmotor (54) angetrieben wird, um Gas über eine Leitung (12) in die Kammer zu pumpen, und ein Heizgerät (70), das die Leitung (12) umschließt, um das durch sie hindurch von der Pumpe (10) in die Kammer (14) strömende Gas zu erhitzen, wobei das Gehäuse (2) eine Zwischenwand (4) aufweist, die eine erste Kammer (6) für die Pumpe und eine zweite Kammer (8) für den Motor begrenzt.

7. Vorrichtung nach Anspruch 1, wobei das Ausströmrohr (80) flexibel ist und sein gegenüberliegendes Ende mit einer Abdeckung (84), die ein Paar Durchgänge (86, 88) hat, versehen ist, um den erhitzten Strom zur Verwendung an zwei Brennermündungen eines Benutzers in zwei Ströme aufzuspalten.

8. Vorrichtung nach Anspruch 1, wobei der Ultraschallerzeuger (74) ein Piezokristall ist.

9. Vorrichtung nach Anspruch 1, wobei die Gaszuführeinrichtung zum Zuführen eines Stroms heißen Gases zum Aufprallen auf den Flüssigkeitsstrahl (76) in der Kammer (14) eine Umlaufpumpe (10) aufweist, die das Gas über eine Leitung (12) in die Kammer (14) pumpt, und ein die Leitung (12) umschließendes Heizgerät (70), um das durch sie hindurch von der Pumpe (10) in die Kammer (14) strömende Gas zu erhitzen; und wobei der Kammerauslaß (78) einen Temperaturfühler (90) enthält zum Erfühlen der Temperatur dort und zum Selbstregeln des Heizgeräts (70), um eine relativ konstante Temperatur am Kammerauslaß (78) aufrechtzuerhalten.

FIG.1

FIG. 2

FIG. 5

FIG. 3

FIG. 4